# EUROPEAN PATENT SPECIFICATION

(11) **EP 3 777 652 B1**
(45) Date of publication and mention of the grant of the patent: **10.09.2025**
(21) Application number: 19774949.2
(22) Date of filing: 28.02.2019
(51) Int. Cl.: A61B 3/113, A61B 3/10, G06F 3/01

(54) **INFORMATION DETECTION DEVICE, IMAGE PROJECTION DEVICE, INFORMATION DETECTION METHOD, AND IMAGE PROJECTION METHOD**
INFORMATIONSERFASSUNGSVORRICHTUNG, BILDPROJEKTIONSVORRICHTUNG, INFORMATIONSERFASSUNGSVERFAHREN UND BILDPROJEKTIONSVERFAHREN
DISPOSITIF DE DÉTECTION D'INFORMATIONS, DISPOSITIF DE PROJECTION D'IMAGE, PROCÉDÉ DE DÉTECTION D'INFORMATIONS ET PROCÉDÉ DE PROJECTION D'IMAGE

(30) Priority: 26.03.2018 JP 2018058483
(43) Date of publication of application: 17.02.2021
(73) Proprietor: Sony Group Corporation, Tokyo 108-0075 (JP)
(72) Inventor: MAMISHIN, Yuki, Tokyo 108-0075 (JP)
(74) Representative: D Young & Co LLP
(86) International application number: PCT/JP2019/007878
(87) International publication number: WO 2019/187958

(56) References cited:
- JP-A- 2008 000 343
- US-A1- 2010 060 551
- US-A1- 2016 081 547
- EIJI SHIMIZU: "Retinal Scanning/Projection Display", THE JOURNAL OF THE INSTITUTE OF IMAGE INFORMATION AND TELEVISION ENGINEERS, vol. 65, no. 6, 1 January 2011 (2011-01-01), pages 758 - 763, XP009515367, ISSN: 1342-6907, DOI: 10.3169/itej.65.758

## Description

### Technical Field

The present technology relates to an information detection apparatus, a video projection apparatus, an information detection method, and a video projection method. More specifically, the present technology relates to an information detection apparatus, a video projection apparatus, an information detection method, and a video projection method that are capable of detecting a pupil position.

### Background Art

In recent years, attention has been paid to the technology of displaying a video superimposed on an outside scene such as a real scene. This technology is also referred to as augmented reality (AR) technology. One example of a product using this technology is a head-mounted display. The head-mounted display is used by being worn on a user's head. In an image display method using the head-mounted display, for example, the eyes of the user are irradiated with light from the head-mounted display in addition to light from the outside, whereby a video is superimposed on an image of the outside for display.

In order to present a video to the user by the head-mounted display, a line-of-sight direction or a pupil position of the user may be grasped. Regarding the technology for grasping a line-of-sight direction or a pupil position of a user, for example, Patent Literature 1 below describes a scanning display device characterized by incorporating a line-of-sight detecting device for detecting a line-of-sight direction of a user while sharing an optical system of the scanning display device. In addition, a pupil detection device described in Patent Literature 2 below includes a detection unit that detects, as a reflected light flux intensity signal, the intensity of a light flux reflected on the surface of an eyeball among light fluxes incident on the surface of the eyeball, and a processing unit that obtains the position of the pupil on the basis of a change in the intensity of the reflected light flux represented by an intensity signal output from the detection unit. In addition, a focal length control device for a camera described in Patent Literature 3 below is characterized by including, in a camera including line-of-sight detecting means for detecting which part of the viewfinder a photographer is looking at on the basis of the line-of-sight direction of the photographer, means for confirming the line-of-sight direction of the photographer in the viewfinder field detected by the line-of-sight detecting means and for setting a focal length value of the camera on the basis of a result of the confirmation.

Patent literature 4 discloses a beam scanning-type display device used as a head-mounted display or a head-up display includes a light source which emits a beam, a scanning unit which performs scanning using the beam emitted from the light source, a deflecting unit which deflects the beam used for the scanning by the scanning unit in the direction toward an eye of a user, and a wavefront shape changing unit which changes the wavefront shape of the beam from the light source so that the beam spot size falls within the predetermined allowable range, and emits the beam to the wavefront shape changing unit . Patent literature 5 discloses an ophthalmic apparatus which substantially adjusts a detecting optical axis in a direction of a visual axis even outside of the region while minimizing an effective aperture of an imaging lens for an ocular fundus high magnification observation and images an ocular fundus at a high magnification/high resolution. The ophthalmic apparatus includes an ocular fundus imaging system and a control part which detects the direction of the visual axis of an eye to be examined and performs tracking on the ocular fundus of the eye to be examined from the detected result of the direction of the visual axis, the control part includes: an optical system, in which scan mirrors for scanning a region where a detecting luminous flux projected on an ocular fundus of the eye to be examined or reflected light from the ocular fundus is detected, tracking mirrors for controlling the tracking for the ocular fundus, and an objective lens placed to face the eye to be examined are sequentially arranged. Patent literature 6 discloses an eye tracking and gaze fixation detection system that includes an electronically scannable optical illumination system that emits polarized near-infrared (NIR) light to a retina in an eye of a subject. Non-patent literature 1 discloses the operating principle, system configuration, and operating characteristics of a retinal scanning / projection display.Citation List

### Patent Literature

Patent Literature 1: Japanese Patent Application Laid-Open No. 2003-029198
Patent Literature 2: Japanese Patent Application Laid-Open No. 2006-058505
Patent Literature 3: Japanese Patent Application Laid-Open No. HEI 05-317260
Patent literature 4: US 2010/060551 A
Patent literature 5: JP 2008/000343 A
Patent literature 6 US 2016/081547 A1

Non-patent literature 1: Eiji Shimizu: "Retinal Scanning/Projection Display", The journal of the institute of image information and television engineers, vol. 65, no. 6, 1 January 2011 (2011-01-01), pages 758-763, DOI: 10.3169itej.65.758

### Disclosure of Invention

### Technical Problem

The head-mounted display is used by being worn on the head of a user and is thus expected for further miniaturization. The head-mounted display is also expected for further reduction in power consumption.

It is an object of the present technology to provide a new technique for detecting information such as a line-of-sight direction or a pupil position. In particular, it is an object of the present technology to reduce the size and/or reduce the power consumption of an apparatus for detecting such information. Solution to Problem

The present technology provides an information detection apparatus, including: an irradiation unit that irradiates an eyeball with light; a scanning mirror that scans reflected light from the eyeball; and a detection unit that detects the reflected light scanned by the scanning mirror. Particular and preferred aspects of the invention are set out in the independent and dependent claims.

According to the invention, the information detection apparatus includes a controller that performs estimation processing for a pupil position or a line-of-sight direction of the eyeball on the basis of the reflected light detected by the detection unit.

According to an example of the present technology, the controller may estimate the pupil position or the line-of-sight direction of the eyeball on the basis of a scanning oscillation angle of the scanning mirror and the reflected light detected by the detection unit.

According to an example of the present technology, the controller may estimate the pupil position or the line-of-sight direction of the eyeball on the basis of a scanning oscillation angle of the scanning mirror and an intensity of the reflected light detected by the detection unit.

According to the invention, the irradiation unit is configured to irradiate the eyeball with the light directly without the scanning mirror.

According to an example of the present technology, the light for irradiating the eyeball may be non-visible light.

According to an example of the present technology, the light for irradiating the eyeball may be infrared light.

According to an example of the present technology, the light for irradiating the eyeball may be beam-like light.

In addition, the present technology provides a video projection apparatus including: an irradiation unit that irradiates an eyeball with light; a scanning mirror that scans reflected light from the eyeball; a detection unit that detects the reflected light scanned by the scanning mirror; a controller that performs estimation processing for a pupil position of the eyeball on the basis of the reflected light detected by the detection unit; and a video display light irradiation unit that irradiates the pupil position estimated by the controller with video display light such that the video display light passes through the pupil position.

According to an example of the present technology, the video display light may be condensed in a vicinity of a pupil, and a retina may be irradiated with the video display light.

According to an example of the present technology, the eyeball may be irradiated with the video display light via the scanning mirror.

According to an example of the present technology, the video projection apparatus may be an eyewear display.

In addition, the present technology provides an information detection method including: irradiating an eyeball with light; scanning reflected light from the eyeball by a scanning mirror; and detecting the reflected light scanned by the scanning mirror.

In addition, the present technology provides a video projection method including: irradiating an eyeball with light; scanning reflected light from the eyeball by a scanning mirror; detecting the reflected light scanned by the scanning mirror; estimating a pupil position of the eyeball on the basis of the reflected light detected in the detection step; and irradiating the pupil position estimated in the estimation step with video display light such that the video display light passes through the pupil position. Advantageous Effects of Invention

In the present technology, the reflected light from the eyeball is detected via the scanning mirror. Thus, the size and power consumption of the detection unit can be reduced.

Note that the effects exerted by the present technology are not necessarily limited to the effects described herein, and any of the effects described herein may be exerted.

### Brief Description of Drawings

[Fig. 1] Fig. 1 is a schematic diagram of an information detection apparatus according to the present technology.
[Fig. 2] Fig. 2 is a diagram showing an example of a scanning line of a scanning mirror.
[Fig. 3] Fig. 3 is a schematic diagram of an information detection apparatus according to the present technology.
[Fig. 4] Fig. 4 is a schematic diagram of a video projection apparatus according to the present technology.
[Fig. 5] Fig. 5 is a schematic diagram of a video projection apparatus according to the present technology.
[Fig. 6] Fig. 6 is a schematic diagram of a video projection apparatus according to the present technology.
[Fig. 7] Fig. 7 is a diagram showing an example of a flow of an information detection method according to the present technology.
[Fig. 8] Fig. 8 is a diagram showing an example of a flow of a video projection method according to the present technology.
[Fig. 9] Fig. 9 is a diagram showing an example of a head-mounted display according to the present technology.
[Fig. 10] Fig. 10 is a diagram showing an example of a head-mounted display according to the present technology.
[Fig. 11] Fig. 11 is a diagram showing a configuration example of an information detection apparatus according to the present technology.
[Fig. 12] Fig. 12 is a graph showing a relationship between light intensity and a scanning oscillation angle.
[Fig. 13] Fig. 13 is a diagram showing a configuration example of an information detection apparatus according to the present technology.
[Fig. 14] Fig. 14 is a graph showing a relationship between the light intensity and the scanning oscillation angle.
[Fig. 15] Fig. 15 is a diagram showing a configuration example of an information detection apparatus according to the present technology.
[Fig. 16] Fig. 16 is a graph showing a relationship between the light intensity and the scanning oscillation angle.
[Fig. 17] Fig. 17 is a diagram showing a configuration example of a video projection apparatus according to the present technology.
[Fig. 18] Fig. 18 is a diagram showing a configuration example of a video projection apparatus according to the present technology.
[Fig. 19] Fig. 19 is a diagram showing a configuration example of a video projection apparatus according to the present technology.

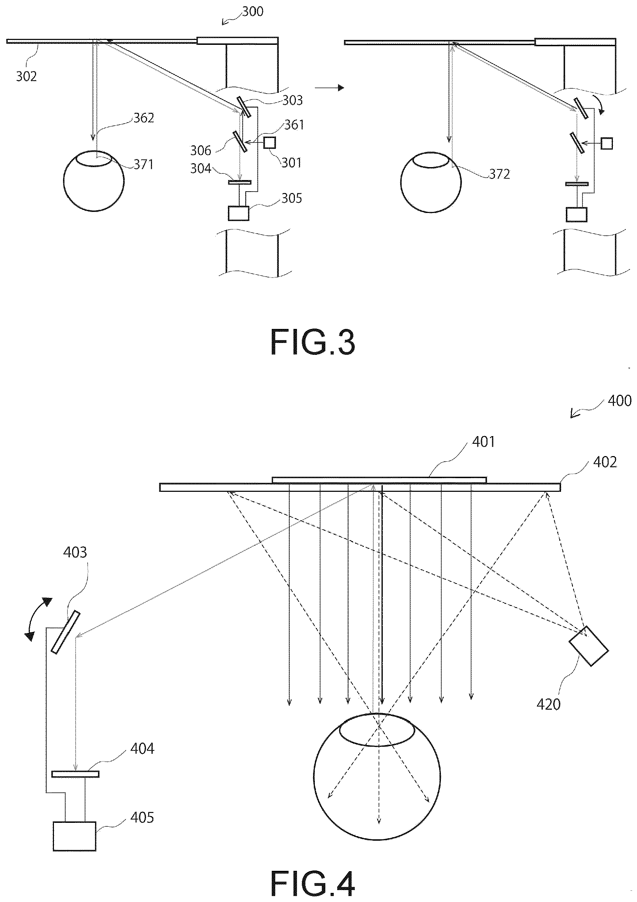

Figures 3, 5, 6, 9, 10, 13, 14, 15, 16, 18, 19 as described herein are incorporated as background information and do not form part of the invention. Mode(s) for Carrying Out the Invention

Hereinafter, suitable embodiments for carrying out the present technology will be described. Note that the embodiments to be described below show representative embodiments of the present technology. Note that the description of the present technology will be given in the following order.
1. First Embodiment (Information Detection Apparatus)
   (1) Description of First Embodiment
   (2) Example of First Embodiment (Information Detection Apparatus)
   (3) Second Example (the second example is incorporated as background information and does not form part of the invention) (Information Detection Apparatus)
2. Second Embodiment (Video Projection Apparatus)
   (1) Description of Second Embodiment
   (2) Example of Second Embodiment (Video Projection Apparatus)
   (3) Third Example (Video Projection Apparatus) (the third example is incorporated as background information and does not form part of the invention)
   (4) Fourth Example (Video Projection Apparatus) (the fourth example is incorporated as background information and does not form part of the invention)
3. Third Embodiment (Information Detection Method)
   (1) Description of Third Embodiment
   (2) Example of Third Embodiment (Information Detection Method)
4. Fourth Embodiment (Video Projection Method)
   (1) Description of Fourth Embodiment
   (2) Example of Fourth Embodiment (Video Projection Method)
5. Configuration Examples of Apparatus
6. Examples
   (1) Example 1 (Simulation of Example of First Embodiment)
   (2) Example 2 (Simulation of Second Example)
   (3) Example 3 (Simulation of Second Example)
   (4) Example 4 (Simulation of Example of Second Embodiment)
   (5) Example 5 (Simulation of Third Example)
   (6) Example 6 (Simulation of Fourth Example)

### 1. First Embodiment (Information Detection Apparatus)

### (1) Description of First Embodiment

An information detection apparatus according to the present technology includes an irradiation unit that irradiates an eyeball with light, a scanning mirror that scans reflected light from the eyeball, and a detection unit that detects the reflected light scanned by the scanning mirror. That is, the reflected light from the eyeball reaches the detection unit via the scanning mirror. As a result, information regarding the eyeball, for example, information regarding a line-of-sight direction, a pupil position, or the like can be detected on the basis of the detected reflected light.

In addition, since the reflected light from the eyeball reaches the detection unit via the scanning mirror as described above, the scanning mirror is driven, and thus the reflected light from various positions of the eyeball can be detected by, for example, one light detection element. Thus, it is possible to reduce the size of the detection unit. In addition, since the number of light detection elements constituting the detection unit of the apparatus according to the present technology may be small, the apparatus has less power consumption.

In addition, as described above, since the reflected light from the eyeball reaches the detection unit via the scanning mirror, a noise component such as ambient light or stray light can be eliminated.

In the line-of-sight detecting device described in Patent Literature 1, for example, four infrared sensors are provided on a concave mirror in front of the eye in order to detect infrared rays reflected on the eyeball. Each of the plurality of infrared sensors detects infrared rays reflected on the eyeball. The device then detects the position of the eyeball on the basis of a plurality of detected values. However, in such a detection method, the detection accuracy is not sufficient in some cases. In addition, since the device includes the plurality of infrared sensors, the power consumption thereof is large.

In the information detection apparatus according to the present technology, the light reflected on the eyeball reaches the detection unit via the scanning mirror. That is, the information detection apparatus according to the present technology can detect reflected light from various positions of the eyeball. Thus, the information detection apparatus according to the present technology has high detection accuracy. In addition, the information detection apparatus according to the present technology can detect reflected light from various positions of the eyeball by using, for example, one light detection element by driving the scanning mirror. Thus, the information detection apparatus according to the present technology has small power consumption.

The pupil detection device described in Patent Literature 2 described above scans infrared rays and irradiates the eye with the infrared rays. Patent Literature 2 discloses a pupil detection unit that receives infrared light reflected on an eye through a condensing lens. However, when receiving the infrared light through the condenser lens, the device is susceptible to ambient light or stray light.

Also in the focal length control device described in Patent Literature 3, infrared light reflected on the eyeball is condensed by a light receiving lens. Thus, the device is also susceptible to ambient light or stray light.

In a case where light detection is performed using the information detection apparatus according to the present technology, the reflected light from the eyeball reaches the detection unit via the scanning mirror. That is, the angle of the light reaching the detection unit is limited. Thus, using the information detection apparatus according to the present technology can lead to a reduction in the influence of a noise component such as ambient light or stray light, for example.

### (2) Example of First Embodiment (Information Detection Apparatus)

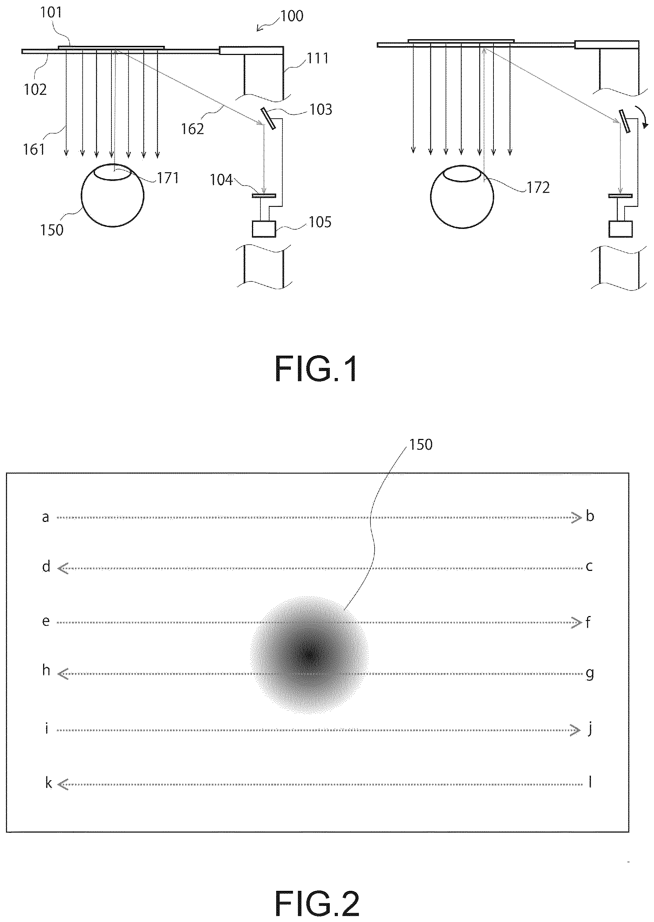

Hereinafter, an example of the information detection apparatus according to the present technology will be described with reference to Fig. 1. Fig. 1 is a schematic diagram of the information detection apparatus according to the present technology. In the schematic diagram, the traveling direction of light for performing information detection by using the information detection apparatus is indicated by arrows.

As shown in Fig. 1, an information detection apparatus 100 includes an irradiation unit 101, a light guide unit 102, a scanning mirror 103, a detection unit 104, and a controller 105.

The irradiation unit 101 irradiates an eyeball 150 with light 161. The light 161 to be emitted is used to detect information regarding the eyeball. The light 161 to be emitted is favorably non-visible light, more favorably infrared light. If the light to be emitted is non-visible light, especially infrared light, the burden on the eye at the time of information detection is reduced. In addition, such light reduces the impact on an outside scene or video recognized by the user.

For example, if the information detection apparatus 100 is a glasses-like apparatus, for example, the irradiation unit 101 may be attached to any position of a rim or lens of the glasses-like apparatus or may be formed as a part of the rim or lens.

The irradiation unit 101 may be provided such that the light 161 passes through the light guide unit 102 and reaches the eyeball 150. For example, the irradiation unit 101 may be provided on the surface opposite to the eyeball-side surface of the rim or the lens of the glasses-like apparatus. Alternatively, the irradiation unit 101 may be provided such that the light 161 reaches the eyeball 150 without passing through the light guide unit 102. In this case, for example, the irradiation unit 101 may be provided on the eyeball side of the rim or lens of the glasses-like apparatus. The irradiation unit 101 may be, for example, a planar light source.

The eyeball is favorably irradiated with the light 161 as parallel light. This allows more accurate information detection. For example, in a case where the irradiation unit 101 is configured such that the light 161 passes through the light guide unit 102 and reaches the eyeball 150, the light guide unit 102 may collimate the light 161. Alternatively, for example, in a case where the irradiation unit 101 is provided such that the light 161 reaches the eyeball 150 without passing through the light guide unit 102, the irradiation unit 101 itself may be configured to output the light 161 as parallel light.

The eyeball 150 is irradiated with the light 161 emitted by the irradiation unit 101 only through the light guide unit 102. That is, the eyeball 150 is irradiated with the light 161 without the scanning mirror 103. Thus, the emitted light 161 is not affected by the reflectance of the scanning mirror 103.

The light guide unit 102 may have a characteristic of transmitting the light 161 emitted from the irradiation unit 101. In addition, the light guide unit 102 may have a characteristic of reflecting reflected light 162 from the eyeball toward the scanning mirror 103. Examples of the light guide unit having such characteristics may include a holographic optical element.

In a case where the light emitted from the irradiation unit 101 is not parallel light, the light guide unit 102 may favorably have an optical characteristic of collimating the light.

For example, in a case where the information detection apparatus 100 is a glasses-like apparatus, for example, the light guide unit 102 may be a lens itself of the glasses-like apparatus or may be configured as a part of the lens.

The scanning mirror 103 may be, for example, a micro-electro-mechanical system (MEMS) mirror. The scanning mirror 103 scans the reflected light 162 from the eyeball, which is reflected by the light guide unit 102. The scanning mirror 103 is driven to change the orientation of the surface of the scanning mirror 103, and thus the reflected light 162 reflected at various positions of the eyeball 150 is reflected by the scanning mirror 103 and proceeds to the detection unit 104.

The scanning mirror 103 is configured to cause the reflected light 162 reflected by the light guide unit 102 to reach the detection unit 104. In order to cause the reflected light 162 reflected by the light guide unit 102 to reach the scanning mirror 103, an optical system may be appropriately provided on the path of the reflected light between the light guide unit 102 and the scanning mirror 103. In addition, in order to cause the reflected light 162 reflected by the scanning mirror 103 to reach the detection unit 104, an optical system may be appropriately provided also on the path of the reflected light between the scanning mirror 103 and the detection unit 104.

For example, in a case where the information detection apparatus 100 is a glasses-like apparatus, the scanning mirror 103 may be attached to any position of a temple 111 of the glasses-like apparatus or may be provided so as to be included in the temple.

In the present technology, scanning may include guiding the reflected light, which is reflected at various reflection positions of an eyeball, to a predetermined light detection element by the scanning mirror. For example, in the present technology, scanning includes guiding the reflected light, which is reflected at each reflection position from a certain point of the eyeball to another point thereof, to a light detection element by driving the scanning mirror. The reflection position of the reflected light guided to the light detection element may be moved onedimensionally or may also be moved two-dimensionally. Favorably, the reflection position of the reflected light guided to the light detection element may be moved two-dimensionally.

This allows the information regarding the eyeball to be more accurately grasped.

Fig. 2 shows an example of a scanning line of the scanning mirror 103. As shown in Fig. 2, the position of the reflected light scanned by the scanning mirror moves from a position "a" to a position "b" by driving the scanning mirror 103. The position of the reflected light scanned by the scanning mirror then moves from a position "c" to a position "d". Similarly, the position of the reflected light scanned by the scanning mirror moves from a position "e" to a position "f", from a position "g" to a position "h", from a position "i" to a position "j", and from a position "k" to a position "l". In such a manner, the reflection position of the reflected light scanned by the scanning mirror 103 may be moved two-dimensionally. The reflected light reflected on the eyeball (or pupil) 150 is detected on the scanning line from the position "e" to the position "f" and on the scanning line from the position "g" to the position "h". In response to a change in the position of the eyeball 150, the scanning line on which the reflected light reflected on the eyeball 150 is detected, or the position on the scanning line may be changed.

The detection unit 104 detects the reflected light 162 reflected by the scanning mirror 103. The detection unit 104 includes, for example, a photodiode. The type of the detection unit 104 may be appropriately selected by a person skilled in the art depending on the type of light to be detected. For example, if the reflected light 162 is infrared light, the detection unit 104 includes an infrared sensor.

The detection unit 104 may detect the intensity of the reflected light 162, for example. The intensity of the reflected light 162 differs depending on the position of the eyeball 150 on which the reflected light 162 is reflected. For example, the intensity of the reflected light reflected at the pupil portion is different from the intensity of the reflected light reflected at the iris portion. In addition, the intensity of the reflected light reflected on the surface (e.g., cornea) of the eyeball is stronger than the intensity of scattered light reflected on the fundus (e.g., retina) of the eyeball. Thus, the pupil position and/or the line-of-sight direction can be estimated on the basis of the orientation of the surface of the scanning mirror 103 or the scanning oscillation angle and the light intensity detected by the detection unit 104.

In addition, in another embodiment, the detection unit 104 may generate an image on the basis of the detected reflected light 162. For example, the detection unit 104 may include an image sensor such as a complementary metal-oxide-semiconductor (CMOS) or charge-coupled device (CCD), particularly an infrared image sensor, in order to generate an image based on the reflected light 162. For example, the controller 105 performs image processing on the image generated on the basis of the reflected light 162, and thus information regarding the eyeball (e.g., pupil position or line-of-sight direction) may be acquired.

The detection unit 104 may be connected to the controller 105. Information regarding the light detected by the detection unit 104, such as intensity, may be processed by the controller 105.

For example, in a case where the information detection apparatus 100 is a glasses-like apparatus, the detection unit 104 may be attached to any position of the temple of the glasses-like apparatus or may be provided so as to be included in the temple.

The controller 105 acquires the information regarding the eyeball (e.g., pupil position, line-of-sight direction, or rotation angle of the eyeball) on the basis of the reflected light (e.g., intensity of the reflected light) detected by the detection unit 104. The controller 105 performs estimation processing for the pupil position or the line-of-sight direction of the eyeball on the basis of the reflected light.

In addition, the controller 105 may be connected to the scanning mirror 103 so as to be capable of controlling the scanning mirror 103. For example, the controller 105 may drive the scanning mirror 103 within a predetermined scanning oscillation angle. The controller 105 can acquire information regarding the scanning mirror 103, for example, information regarding the orientation of the surface of the scanning mirror, the scanning oscillation angle, or the like.

Favorably, the controller 105 may acquire information regarding the eyeball on the basis of the information regarding the scanning mirror 103 and the information regarding the reflected light detected by the detection unit 104. For example, the controller 105 may perform estimation processing for, for example, the pupil position, the line-of-sight direction, the rotation angle of the eyeball, or the like on the basis of the scanning oscillation angle of the scanning mirror 103 and the reflected light (particularly, the intensity of the reflected light) detected by the detection unit 104.

The controller 105 may include a processor such as a central processing unit (CPU) and a memory such as a random access memory (RAM) and/or a read-only memory (ROM). The memory may store a program or the like for causing the apparatus to execute an information detection method or a video projection method according to the present technology. The processor may achieve the functions of the controller 105.

The controller 105 may control the irradiation of light by the irradiation unit 101. For example, the controller 105 may switch on or off the irradiation of light by the irradiation unit 101, or may change the intensity or type of light to be emitted. In addition, the controller 105 may control the driving of the scanning mirror 103. For example, the controller 105 may change the scanning oscillation angle of the scanning mirror 103. In addition, the controller 105 may control detection performed by the detection unit 104. For example, the controller 105 may switch on or off the detection operation performed by the detection unit 104.

For example, in a case where the information detection apparatus 100 is a glasses-like apparatus, the controller 105 may be provided so as to be included in a temple of the glasses-like apparatus. Alternatively, the controller 105 may be provided in an apparatus different from the glasses-like apparatus and may be connected to the glasses-like apparatus by wire or wireless.

For example, if the surface of the scanning mirror 103 is oriented in a direction as shown on the left in Fig. 1, the reflected light (including, e.g., surface reflected light or corneal reflected light) passing through a position 171 on the eyeball 150 is reflected by the scanning mirror 103 and detected by the detection unit 104. If the orientation of the surface of the scanning mirror 103 is changed to the direction shown on the right in Fig. 1, the reflected light (e.g., scattered light reflected on the iris) passing through a position 172 on the eyeball 150 is reflected by the scanning mirror 103 and detected by the detection unit 104. The intensity of the reflected light passing through the position 171 and the intensity of the reflected light passing through the position 172 are different from each other. In such a manner, the reflection position on the eyeball 150 of the reflected light reaching the detection unit 104 differs depending on the orientation of the surface of the scanning mirror 103. Thus, if the scanning mirror 103 is driven, information regarding the reflected light reflected at various positions on the eyeball 150 can be acquired.

The acquired information regarding the reflected light corresponds to, for example, the orientation of the surface of the scanning mirror 103 or the scanning oscillation angle. Thus, the information regarding the eyeball 150 can be acquired on the basis of the acquired information regarding the light, and the orientation of the surface of the scanning mirror 103 or the scanning oscillation angle.

In addition, unlike the case of an information detection apparatus 300 to be described below in "(3) Second Example (Information Detection Apparatus)", the light from the irradiation unit 101 of the information detection apparatus 100 is emitted onto the eyeball without the scanning mirror or a half mirror. Thus, the utilization efficiency of the light from the irradiation unit 101 of the information detection apparatus 100 can be made higher than that of light from an irradiation unit 301 of the information detection apparatus 300. In addition, the information detection apparatus 100 is not easily affected by stray light caused by the scanning mirror or the half mirror.

### (3) Second Example (Information Detection Apparatus)

Hereinafter, another example of the information detection apparatus according to the present technology will be described with reference to Fig. 3. Fig. 3 is a schematic diagram of an information detection apparatus according to the present technology. In the schematic diagram, the traveling direction of light for performing information detection by using the information detection apparatus is indicated by an arrow.

As shown in Fig. 3, an information detection apparatus 300 includes an irradiation unit 301, a light guide unit 302, a scanning mirror 303, a detection unit 304, and a controller 305. The information detection apparatus 300 further includes a half mirror 306. Of these constituent elements, the scanning mirror 303, the detection unit 304, and the controller 305 are the same as the scanning mirror 103, the detection unit 104, and the controller 105 described above in "(2) Example of First Embodiment (Information Detection Apparatus)". The information detection apparatus 100 is configured to irradiate the eyeball with the light emitted by the irradiation unit 101 without the scanning mirror 103, whereas the information detection apparatus 300 irradiates the eyeball with light emitted by the irradiation unit 301 via the scanning mirror 303. Thus, the irradiation unit 301 and the light guide unit 302 will be mainly described below.

The irradiation unit 301 is configured to irradiate the eyeball with light 361 via the scanning mirror 303. That is, the light 361 is scanned and emitted to the eyeball.

Favorably, the light 361 may be emitted from the irradiation unit 301 as beam-like light (in particular, laser beam). More favorably, the irradiation unit 301 emits beam-like infrared light. The irradiation unit 301 may emit infrared laser light, for example. This makes it possible to more clearly detect the reflected light coming from the pupil portion, for example. Thus, the pupil position can be estimated more accurately.

The light 361 emitted by the irradiation unit 301 is reflected by the half mirror 306 to reach the scanning mirror 303, further reflected by the scanning mirror 303 to reach the light guide unit 302, and further reflected by the light guide unit 302 to reach the eyeball.

For example, in a case where the information detection apparatus 300 is a glasses-like apparatus, the irradiation unit 301 may be attached to any position of a temple of the glasses-like apparatus. Alternatively, the irradiation unit 301 may be provided so as to be included in the temple. In addition, the irradiation unit 301 may be connected to the controller 305 so as to be controllable by the controller 305.

The half mirror 306 may have a characteristic of reflecting the light from the irradiation unit 301 and transmitting the reflected light from the eyeball. The detection unit 304 detects reflected light 362 reflected by the scanning mirror 303 and transmitted through the half mirror 306.

The light guide unit 302 may have a characteristic of reflecting the light 361 emitted from the irradiation unit 301 and reflecting the reflected light 362 from the eyeball. The light guide unit 302 reflects the reflected light 362 from the eyeball toward the scanning mirror 303. Examples of the element having the above characteristics may include a holographic optical element.

The light guide unit 302 may be, for example, a grating or a grating lens. In a case where the light guide unit 302 is a grating, the light guide unit 302 can selectively reflect only light having a predetermined wavelength. In a case where the light guide unit 302 is a grating lens, the light guide unit 302 can selectively reflect only light having a predetermined wavelength and condense the light toward the eyeball.

In addition, the information detection apparatus 300 may further include another optical element such as a collimator lens between the scanning mirror 303 and the light guide unit 302.

The scanning mirror 303 may be, for example, a MEMS mirror. As described above, the scanning mirror 303 scans the light 361 emitted by the irradiation unit 301 and also scans the reflected light 362 reflected on the eyeball. That is, in the information detection apparatus 300 in this example, the reflected light from the eyeball is scanned by the scanning mirror 303 that scans illumination light.

For example, if the surface of the scanning mirror 303 is oriented in a direction as shown on the left in Fig. 3, the reflected light (e.g., surface reflected light or corneal reflected light) passing through a position 371 on the eyeball is reflected by the scanning mirror 303 and detected by the detector 304. If the orientation of the surface of the scanning mirror 303 is changed to the direction shown on the right in Fig. 1, the reflected light (e.g., scattered light reflected on the iris) passing through a position 372 on the eyeball is reflected by the scanning mirror 303 and detected by the detector 304. The intensity of the reflected light passing through the position 371 and the intensity of the reflected light passing through the position 372 are different from each other. In such a manner, the reflection position on the eyeball of the reflected light reaching the detection unit 304 differs depending on the orientation of the surface of the scanning mirror 303. Thus, if the scanning mirror 303 is driven, information regarding the reflected light reflected at various positions on the eyeball can be acquired.

The acquired information regarding the light corresponds to, for example, the orientation of the surface of the scanning mirror 303 or the scanning oscillation angle. Thus, various types of information regarding the eyeball can be acquired on the basis of the acquired information regarding the light, and the orientation of the surface of the scanning mirror 303 or the scanning oscillation angle.

The information detection apparatus 300 includes a common optical system (e.g., scanning mirror) on the path of the light emitted to the eyeball and the reflected light. Thus, the information detection apparatus 300 can be made smaller than the information detection apparatus 100 described above in "(2) Example of First Embodiment (Information Detection Apparatus)".

In addition, the light from the irradiation unit 301 of the information detection apparatus 300 is emitted to the eyeball via the half mirror 306 and the scanning mirror 303. Thus, the light utilization efficiency may be lower than that of the information detection apparatus 100 described above in "(2) Example of First Embodiment (Information Detection Apparatus)". However, if the light transmittance or reflectance of each of the half mirror 306 and the scanning mirror 303 is increased, the utilization efficiency of the light can be increased.

### 2. Second Embodiment (Video Projection Apparatus)

### (1) Description of Second Embodiment

A video projection apparatus according to the present technology includes a video display light irradiation unit that irradiates a pupil position estimated by the controller with video display light such that the video display light passes through the pupil position, in addition to the constituent elements described above in "1. First Embodiment (Information Detection Apparatus)".

The video projection apparatus according to the present technology exhibits the effects described above in "1. First Embodiment (Information Detection Apparatus)". Furthermore, the video projection apparatus according to the present technology exhibits an effect capable of emitting the video display light to an appropriate position.

### (2) Example of Second Embodiment (Video Projection Apparatus)

Hereinafter, an example of a video projection apparatus according to the present technology will be described with reference to Fig. 4. Fig. 4 is a schematic diagram of a video projection apparatus according to the present technology. In the schematic diagram, the traveling direction of light for performing information detection by the video projection apparatus and the direction of the video display light are indicated by the arrows of the dotted line.

As shown in Fig. 4, a video projection apparatus 400 includes an irradiation unit 401, a light guide unit 402, a scanning mirror 403, a detection unit 404, and a controller 405. The video projection apparatus 400 further includes a video display light irradiation unit 420. Of these constituent elements, the irradiation unit 401, the scanning mirror 403, the detection unit 404, and the controller 405 are the same as the irradiation unit 101, the scanning mirror 103, the detection unit 104, and the controller 105 described above in "(2) Example of First Embodiment (Information Detection Apparatus)" of the Section 1. Thus, the information (e.g., pupil position) as described in "(2) Example of First Embodiment (Information Detection Apparatus)" of the Section 1 can be detected.

The video projection apparatus 400 is an apparatus in which the video display light irradiation unit 420 is added to the information detection apparatus 100 described above in "(2) Example of First Embodiment (Information Detection Apparatus)" of the Section 1. Hereinafter, the light guide unit 402 and the video display light irradiation unit 420 will be mainly described.

The light guide unit 402 may have a characteristic of transmitting illumination light emitted from the irradiation unit 401 and reflecting reflected light from the eyeball. In a case where the light emitted from the irradiation unit 401 is not parallel light, the light guide unit 402 may favorably have an optical characteristic of collimating the light.

Furthermore, the light guide unit 402 may have a characteristic of reflecting the video display light emitted by the video display light irradiation unit 420. The light guide unit 402 favorably reflects the video display light such that the video display light emitted from the video display light irradiation unit 420 is condensed in the vicinity of the pupil and emitted to the retina. That is, the light guide unit 402 may diffract the video display light such that the video display light travels straight through the pupil. This makes it possible to present a video to the user by a so-called Maxwellian view. Thus, a clear video can be presented to the user.

In the present technology, the video display light may be condensed in the vicinity of the pupil, e.g., on the pupil, or may deviate from the pupil by a few mm to a few tens of mm (e.g., 1 mm to 20 mm, particularly 2 mm to 15 mm) in an optical axis direction. Even if the focal point is not on the pupil as in the latter case, the Maxwellian view can be achieved. A shift of the focal point in the optical axis direction allows the user to hardly lose the video even if the video is shifted. More specifically, the video display light may be condensed on the pupil, in a crystalline lens, or between the corneal surface and the pupil.

For example, in a case where the video projection apparatus 400 is a glasses-like apparatus, the light guide unit 102 may be, for example, a lens itself of the glasses-like apparatus. Alternatively, the light guide unit 102 may be configured as a part of a lens.

The video display light irradiation unit 420 emits the video display light toward the light guide unit 402. The video display light to be emitted may be emitted radially. The video display light irradiation unit 420 may be controlled by the controller 405. For example, the controller 405 may control the video display light irradiation unit 420 to emit the video display light such that the video display light is condensed at the pupil position estimated by the controller 405. This makes it possible to more reliably present the video to the user.

The video display light may be light by which a video can be presented to the user by a Maxwellian view, for example. The video display light may be, for example, light emitted by a light-emitting diode (LED) or a cathode-ray tube (CRT).

For example, in a case where the video projection apparatus 400 is a glasses-like apparatus, the video display light irradiation unit 420 may be attached to a temple portion of the glasses-like apparatus, for example. Alternatively, the video display light irradiation unit 420 may be included in the temple portion. A video is presented to the user by the video display light irradiation unit 420, whereby the video is superimposed on an outside scene viewed by the user through the lens of the glasses-like apparatus.

### (3) Third Example (Video Projection Apparatus)

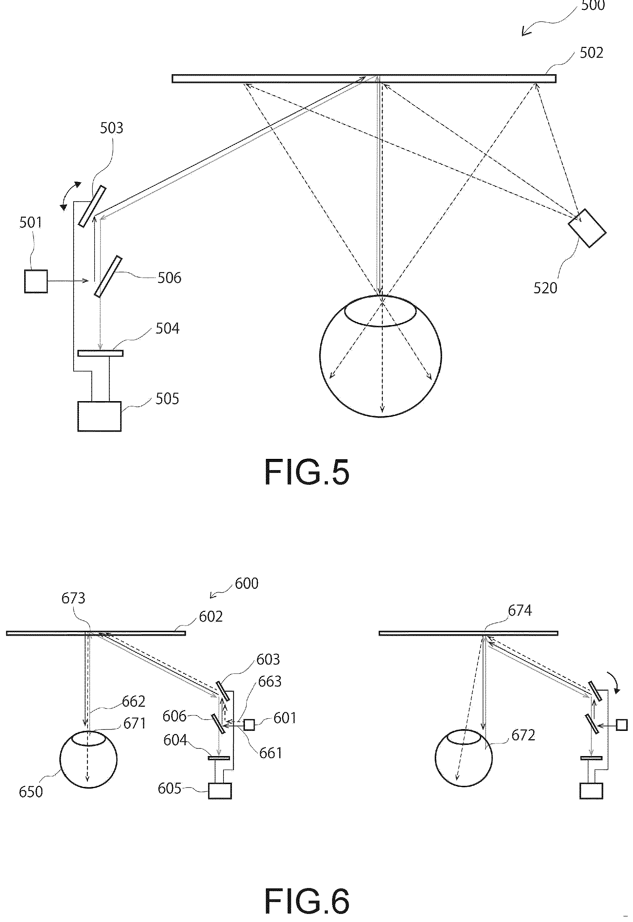

Another example of the video projection apparatus according to the present technology will be described with reference to Fig. 5. Fig. 5 is a schematic diagram of a video projection apparatus according to the present technology. In the schematic diagram, the traveling direction of light for performing information detection according to the present technology and the traveling direction of video display light are indicated by the arrows of the dotted line.

As shown in Fig. 5, a video projection apparatus 500 includes an irradiation unit 501, a light guide unit 502, a scanning mirror 503, a detection unit 504, and a controller 505. The video projection apparatus 500 further includes a half mirror 506 and a video display light irradiation unit 520.

Of these constituent elements, the irradiation unit 501, the light guide unit 502, the scanning mirror 503, the detection unit 504, the controller 505, and the half mirror 506 correspond to the irradiation unit 301, the light guide unit 302, the scanning mirror 303, the detection unit 304, the controller 305, and the half mirror 306 described above in "(3) Second Example (Information Detection Apparatus)" of the Section 1. Thus, the information (e.g., pupil position) as described above in "(3) Second Example (Information Detection Apparatus)" of the Section 1 can be detected.

The video projection apparatus 500 can also be an apparatus in which the video display light irradiation unit 520 is added to the information detection apparatus 300 described above in "(3) Second Example (Information Detection Apparatus)" of the Section 1. Hereinafter, the light guide unit 502 and the video display light irradiation unit 520 will be mainly described.

The light guide unit 502 may have a characteristic of reflecting illumination light emitted from the irradiation unit 501 and reflecting reflected light from the eyeball.

Furthermore, the light guide unit 502 may have a characteristic of reflecting the video display light emitted by the video display light irradiation unit 520. The light guide unit 502 favorably reflects the video display light such that the video display light emitted from the video display light irradiation unit 520 is condensed in the vicinity of the pupil and emitted to the retina. That is, the light guide unit 502 may diffract the video display light such that the video display light travels straight through the pupil. This makes it possible to present a video to the user by a so-called Maxwellian view. Thus, a clear video can be presented to the user.

The video display light irradiation unit 520 emits the video display light toward the light guide unit 502. The video display light to be emitted may be emitted radially. The video display light irradiation unit 520 may be controlled by the controller 505. For example, the controller 505 may control, for example, the video display light irradiation unit 520 to emit the video display light such that the video display light is condensed at the pupil position estimated by the controller 505. This makes it possible to more reliably present the video to the user. The video display light may be light by which a video can be presented to the user by a Maxwellian view, for example. The video display light may be, for example, light emitted by an LED or a CRT.

For example, in a case where the video projection apparatus 500 is a glasses-like apparatus, the video display light irradiation unit 520 may be attached to a temple portion of the glasses-like apparatus, for example. Alternatively, the video display light irradiation unit 520 may be included in the temple portion. A video is presented to the user by the video display light irradiation unit 520, whereby the video is superimposed on an outside scene viewed by the user through the lens of the glasses-like apparatus.

### (4) Fourth Example (Video Projection Apparatus)

Another example of the video projection apparatus according to the present technology will be described with reference to Fig. 6. Fig. 6 is a schematic diagram of a video projection apparatus according to the present technology. In the schematic diagram, the traveling direction of light for performing information detection by the video projection apparatus and the traveling direction of video display light are indicated by the arrows of the dotted line.

As shown in Fig. **6****,** a video projection apparatus 600 includes an irradiation unit 601, a light guide unit 602, a scanning mirror 603, a detection unit 604, and a controller 605. The video projection apparatus 600 further includes a half mirror 606.

The irradiation unit 601 is configured to irradiate an eyeball 650 with illumination light 661 via the scanning mirror 603. That is, the illumination light 661 is scanned and emitted to the eyeball.

Favorably, the illumination light 661 may be emitted from the irradiation unit 601 as beam-like light. More favorably, the irradiation unit 601 emits beam-like infrared light, particularly an infrared laser beam.

The illumination light 661 emitted by the irradiation unit 601 is reflected by the half mirror 606 to reach the scanning mirror 603, further reflected by the scanning mirror 603 to reach the light guide unit 602, and further reflected by the light guide unit 602 to reach the eyeball 650.

The irradiation unit 601 is configured to be capable of emitting video display light 663 in addition to the illumination light 661. For example, the illumination light 661 and the video display light 663 may be output by the irradiation unit 601 in a multiplexed state. A video is presented to the user by the video display light 663.

The irradiation unit 601 may output, for example, laser light in which the video display light and the illumination light are multiplexed, particularly laser light in which red, green, and blue laser light and infrared laser light are multiplexed.

For example, in a case where the video projection apparatus 600 is a glasses-like apparatus, the irradiation unit 601 may be attached to any position of the temple of the glasses-like apparatus. Alternatively, the irradiation unit 601 may be provided so as to be included in the temple. A video is presented to the user by the irradiation unit 601, whereby the video is superimposed on an outside scene viewed by the user through the lens of the glasses-like apparatus.

The light guide unit 602 may have a characteristic of reflecting the illumination light 661 and the video display light 663 emitted from the irradiation unit 601 and reflecting the reflected light 662 from the eyeball. The light guide unit 602 may favorably have a characteristic of reflecting the illumination light 661 to reach the position to be scanned on the eyeball and reflecting the video display light 663 so as to be condensed at the pupil position to irradiate the retina (particularly through the center of the pupil). As the light guide unit having these characteristics, an optical element known in the technical field can be used. Examples of the optical element include a holographic optical element.

For example, as shown on the left in Fig. **6****,** when the illumination light 661 and the video display light 663 reach a position 673 of the light guide unit 602, both the illumination light 661 and the video display light 663 are reflected so as to pass through the center 671 of the pupil. For example, as shown on the right in Fig. **6****,** when the illumination light 661 and the video display light 663 reach a position 674 of the light guide unit 602, the illumination light 661 is reflected so as to be emitted to a position 672 deviated from the center of the pupil, and the video display light 663 is reflected so as to pass through the center 671 of the pupil.

The scanning mirror 603 may be, for example, a MEMS mirror. As described above, the scanning mirror 603 scans the illumination light 661 and the video display light 663 (or combination thereof) emitted by the irradiation unit 601 and also scans the reflected light 662 reflected on the eyeball 650. That is, in the video projection apparatus 600 according to this example, the reflected light from the eyeball and the video display light are also scanned by the scanning mirror that scans the illumination light.

When the eyeball is irradiated with the illumination light, reflected light is generated. When the reflected light is scanned, information regarding the eyeball can be acquired.

In addition, the video display light is scanned on the retina, and thus a video is presented to the user. The video display light is condensed, for example, in the vicinity of the pupil by the scanning and emitted to the retina. That is, the video display light travels straight through the pupil. This makes it possible to present a video to the user by a so-called Maxwellian view. Thus, a clear video can be presented to the user.

For example, if the surface of the scanning mirror 603 is oriented in a direction as shown on the left in Fig. 6, the reflected light (e.g., surface reflected light or corneal reflected light) passing through the center 671 of the eyeball 650 is reflected by the light guide unit 602 and the scanning mirror 603, passes through the half mirror 606, and is detected by the detector 604. If the orientation of the surface of the scanning mirror 603 is changed to the direction shown on the right in Fig. 6, the reflected light (including, e.g., scattered light reflected on the iris) passing through the position 672 of the eyeball 650 is reflected by the light guide unit 602 and the scanning mirror 603, transmitted through the half mirror 606, and detected by the detector 604. In such a manner, the position on the eyeball 650 at which the reflected light reaching the detection unit 604 is reflected differs depending on the orientation of the surface of the scanning mirror 603, and the intensity of the reflected light differs depending on the position on the eyeball at which the reflected light is reflected. Thus, if the scanning mirror 603 is driven, information regarding the reflected light reflected at various positions on the eyeball 650 can be acquired.

The detection unit 604 detects the reflected light 662 reflected by the scanning mirror 603 and transmitted through the half mirror 606.

The controller 605 may process the information regarding the reflected light detected by the detector 604 (e.g., intensity of reflected light). For example, the controller 605 may acquire information regarding the eyeball (e.g., rotation angle of eyeball, pupil position, and line-of-sight direction) on the basis of the information regarding the reflected light. Favorably, the controller 605 may perform estimation processing for the pupil position of the eyeball on the basis of the reflected light detected by the detection unit 604.

In addition, the controller 605 may be configured to be capable of controlling the scanning mirror 603. For example, the controller 605 may drive the scanning mirror 603 within a predetermined scanning oscillation angle. The controller 605 can acquire information regarding the scanning mirror 603, for example, information regarding the orientation of the surface of the scanning mirror, the scanning oscillation angle, or the like.

Favorably, the controller 605 may acquire information regarding the eyeball on the basis of the information regarding the scanning mirror 603 and the information regarding the reflected light detected by the detection unit 604. For example, the controller 605 may estimate the pupil position, the line-of-sight direction, or the rotation angle of the eyeball on the basis of the scanning oscillation angle of the scanning mirror 603 and the reflected light (e.g., intensity of reflected light) detected by the detection unit 604.

In addition, the controller 605 may control the irradiation unit 601 to adjust the video display light to be emitted, on the basis of the acquired information regarding the eyeball. Thus, a video can be displayed at an appropriate position in the field of view of the user.

The controller 605 may include a processor such as a CPU and a memory such as a RAM and/or a ROM. The memory may store a program or the like for causing the apparatus to execute an information detection method or a video projection method according to the present technology. The processor may achieve the functions of the controller 605.

The controller 605 may control the irradiation of the illumination light and/or the video display light by the irradiation unit 601. For example, the controller 605 may switch on or off the irradiation of the illumination light and/or the video display light by the irradiation unit 601. Alternatively, the controller 605 may change the intensity or type of the illumination light and/or the video display light to be emitted. In addition, the controller 605 may control the driving of the scanning mirror 603. For example, the controller 605 may change the scanning oscillation angle of the scanning mirror 603. In addition, the controller 605 may control the detection by the detection unit 604. For example, the controller 605 may switch on or off the detection operation performed by the detection unit 604.

The half mirror 606 may have a characteristic of reflecting the illumination light 661 and the video display light 663 (or combination thereof) and transmitting the reflected light 662. This allows only the reflected light 662 to selectively reach the detection unit 604.

In the video projection apparatus 600, the irradiation unit 601 is configured to be capable of emitting the illumination light and the video display light as described above, and thus the apparatus can be miniaturized. In addition, since the optical system (e.g., scanning mirror) on the path of the illumination light, the video display light, and the reflected light is shared, the apparatus can be further miniaturized.

### 3. Third Embodiment (Information Detection Method)

### (1) Description of Third Embodiment

The present technology also provides an information detection method including: irradiating an eyeball with light; scanning reflected light from the eyeball by a scanning mirror; and detecting the reflected light scanned by the scanning mirror.

The information detection method according to the present technology allows the information of the eyeball as described above in the Section 1 to be detected.

### (2) Example of Third Embodiment (Information Detection Method)

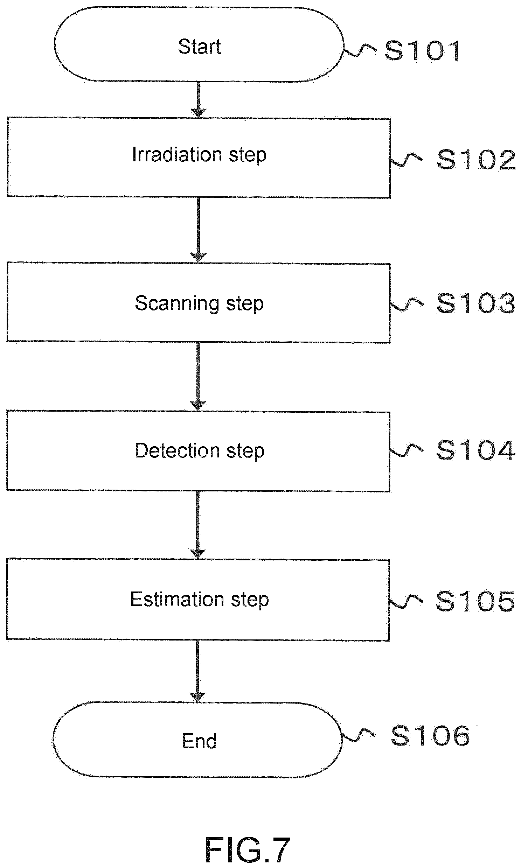

Hereinafter, an example of the information detection method according to the present technology will be described with reference to Figs. 1 and 7. Fig. 7 is a diagram showing an example of a flow of the information detection method according to the present technology.

In Step S101, the information detection apparatus 100 starts information detection processing according to the present technology.

In Step S102, the controller 105 causes the irradiation unit 101 to irradiate an eyeball with illumination light. The light to be emitted is favorably non-visible light, more favorably infrared light. The illumination light may be emitted only when the detection of a pupil position is necessary, for example. Alternatively, the illumination light may be emitted at predetermined time intervals.

In Step S103, the controller 105 drives the scanning mirror 103 to cause the scanning mirror 103 to scan reflected light generated by irradiation of the illumination light onto the eyeball in Step S102. As a result of the scanning, the reflected light reflected at each of various positions of the eyeball proceeds to the detection unit 104.

In Step S104, the controller 105 causes the detection unit 104 to detect the reflected light scanned in Step S103. As a result of the detection, information regarding the reflected light, for example, the intensity of the reflected light, may be acquired.

In Step S105, the controller 105 acquires information regarding the eyeball on the basis of the information regarding the reflected light acquired in Step S104 and the information regarding the scanning in Step S103.

In Step S106, the information detection apparatus 100 terminates the information detection processing according to the present technology.

The above processing may be performed by, for example, the information detection apparatus or the video projection apparatus according to the present technology. For a more detailed operation of each constituent element of these apparatuses in each of the steps described above, refer to "1. First Embodiment (Information Detection Apparatus)" and "2. Second Embodiment (Video Projection Apparatus)" described above.

### 4. Fourth Embodiment (Video Projection Method)

### (1) Description of Fourth Embodiment

The present technology also provides a video projection method including: irradiating an eyeball with light; scanning the reflected light from the eyeball by a scanning mirror; detecting the reflected light scanned by the scanning mirror; estimating a pupil position of the eyeball on the basis of the reflected light detected in the detection step; and irradiating a pupil position estimated in the estimation step with video display light such that the video display light passes through the pupil position.

The video projection method according to the present technology allows the information of the eyeball as described above in the Section 1 to be detected. In addition, the video projection method according to the present technology allows the video display light to be emitted to an accurate position on the basis of the detected information of the eyeball.

### (2) Example of Fourth Embodiment (Video Projection Method)

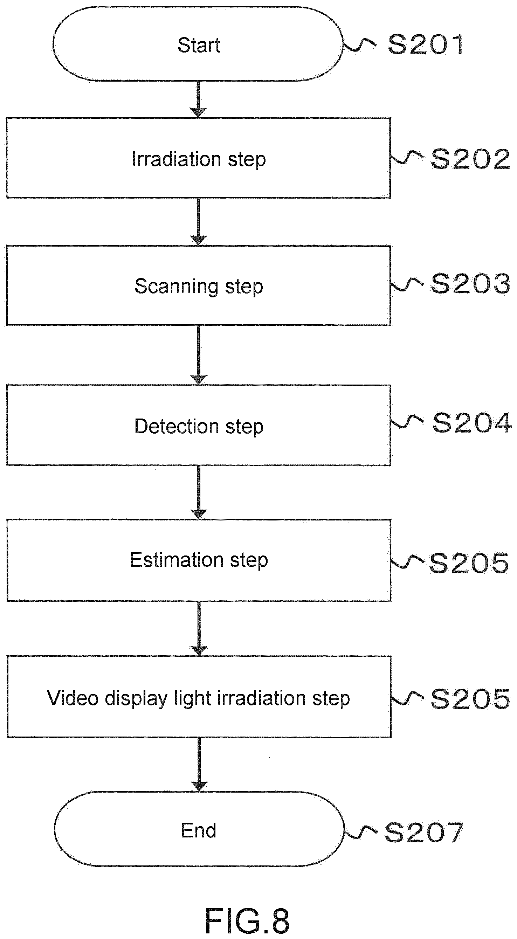

Hereinafter, an example of the video projection method according to the present technology will be described with reference to Figs. 6 and 8. Fig. 8 is a diagram showing an example of a flow of the video projection method according to the present technology.

In Step S201, the video projection apparatus 600 starts video projection processing according to the present technology.

In Step S202, the controller 605 causes the irradiation unit 601 to irradiate an eyeball with illumination light. The light to be emitted is favorably non-visible light, more favorably infrared light. The illumination light may be emitted only when the detection of a pupil position is necessary, for example. Alternatively, the illumination light may be emitted at predetermined time intervals.

In Step S203, the controller 605 drives the scanning mirror 603 to cause the scanning mirror 603 to scan reflected light generated by irradiation of the illumination light onto the eyeball in the Step S202. As a result of the scanning, the reflected light reflected at each of various positions of the eyeball proceeds to the detection unit 604.

In Step S204, the controller 605 causes the detection unit 604 to detect the reflected light scanned in the Step S203. As a result of the detection, information regarding the reflected light, for example, the intensity of the reflected light, may be acquired.

In Step S205, the controller 605 acquires information regarding the eyeball on the basis of the information regarding the reflected light acquired in Step S204 and the information regarding the scanning in Step S203.

In the Step S206, the controller 605 causes the irradiation unit 601 to emit video display light adjusted on the basis of the information (e.g., pupil position) regarding the eyeball acquired in the Step S205. Thus, the eyeball is irradiated with the video display light, and the user can recognize the video.

In Step S207, the video projection apparatus 600 terminates the video projection processing according to the present technology.

The above processing may be performed by, for example, the video projection apparatus according to the present technology. For a more detailed operation of each constituent element of the apparatus in each of the steps described above, refer to "2. Second Embodiment (Video Projection Apparatus)" described above.

### 5. Configuration Examples of Apparatus

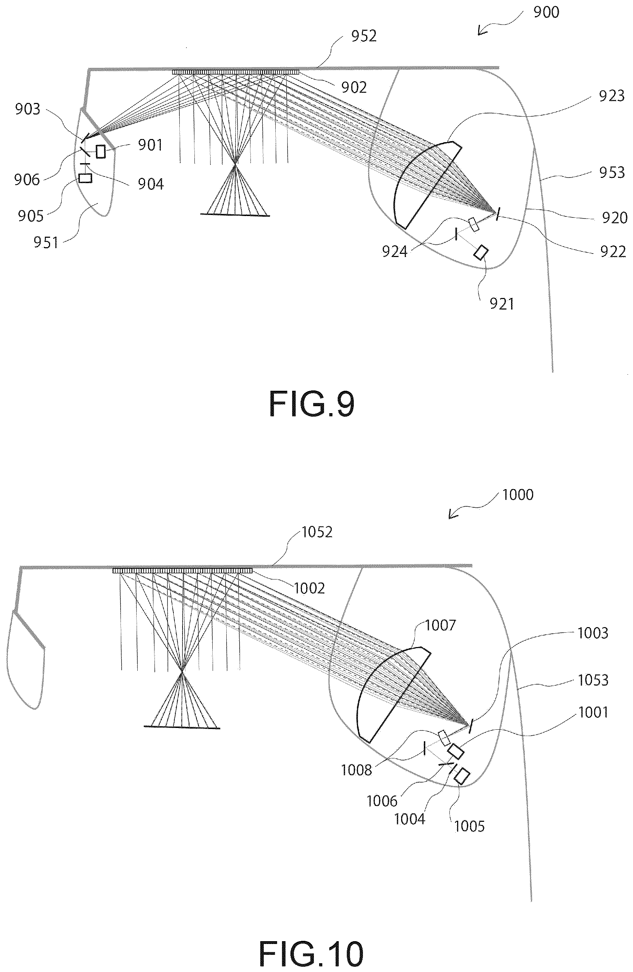

Specific examples of the video projection apparatus according to the present technology will be described below with reference to Figs. 9 and 10.

Fig. 9 shows a head-mounted display (hereinafter, referred to as HMD) 900 as an example of the video projection apparatus according to the present technology. The HMD 900 is a glasses-like apparatus, i.e. an eyewear display.

Main constituent elements for performing the information detection according to the present technology are provided in a nose pad 951 of the HMD 900. That is, in the nose pad 951 of the HMD 900, an irradiation unit 901, a half mirror 906, a scanning mirror 903, a detection unit 904, and a controller 905 are provided. Note that the controller 905 may be provided not in the nose pad 951 but in a temple 953, for example.

Main constituent elements for presenting a video to a user on the basis of information detected according to the present technology are provided in the temple 953 of the HMD 900. That is, a video display light irradiation unit 920 is provided in the temple 953 of the HMD 900. The video display light irradiation unit 920 includes an output unit 921, a scanning mirror 922, a lens 923, and an optical system 924. The video display light irradiation unit 920 may be connected to the controller 905.

In addition, a light guide unit 902 is provided to the lens 952 of the HMD 900.

Illumination light emitted from the irradiation unit 901 is reflected by the half mirror 906 and the scanning mirror 903 and reaches the light guide unit 902. The illumination light is further reflected by the light guide unit 902 and reaches the eyeball. Reflected light from the eyeball is reflected by the light guide 902, scanned by the scanning mirror 903, and transmitted through the half mirror 906. The reflected light transmitted through the half mirror 906 is detected by the detection unit 904. Information regarding the eyeball is detected on the basis of information regarding the detected reflected light and, for example, information regarding the scanning mirror 903. On the basis of the information regarding the eyeball, the video display light irradiation unit 920 emits video display light such that the user can accurately recognize the video.

Fig. 10 shows another example of the HMD as the video projection apparatus according to the present technology. An HMD 1000 shown in Fig. 100 has a glasses-like shape, i.e., can also be an eyewear display.

Main constituent elements for performing the information detection according to the present technology and main constituent elements for presenting a video to a user on the basis of the information detected according to the present technology are provided in a temple 1053 of the HMD 1000. In addition, the HMD 1000 has a common optical system on the path of illumination light to the eyeball, reflected light from the eyeball, and video displaying light. In other words, an irradiation unit 1001, a scanning mirror 1003, a detection unit 1004, and a controller 1005 are provided in the temple 1053 of the HMD 1000. Furthermore, a half mirror 1006, a lens 1007, and an optical system (e.g., reflecting mirror or the like) 1008 are provided in the temple 1053 of the HMD 1000. In addition, a light guide unit 1002 is provided in a lens 1052 of the HMD 1000.

Illumination light emitted from the irradiation unit 1001 reaches the light guide unit 1002 via the half mirror 1006, the optical system 1008, and the scanning mirror 1003. The illumination light is further reflected by the light guide unit 1002 and reaches the eyeball. Reflected light from the eyeball is reflected by the light guide unit 1002, is scanned by the scanning mirror 1003, passes through the optical system 1008, and is transmitted through the half mirror 1006. The reflected light transmitted through the half mirror 1006 is detected by the detection unit 1004. Information regarding the eyeball is detected on the basis of information regarding the detected reflected light and, for example, information regarding the scanning mirror 1003. On the basis of the information regarding the eyeball, the irradiation unit 1001 emits video display light such that the user can accurately recognize the video.

### 6. Examples

### (1) Example 1 (Simulation of Example of First Embodiment)

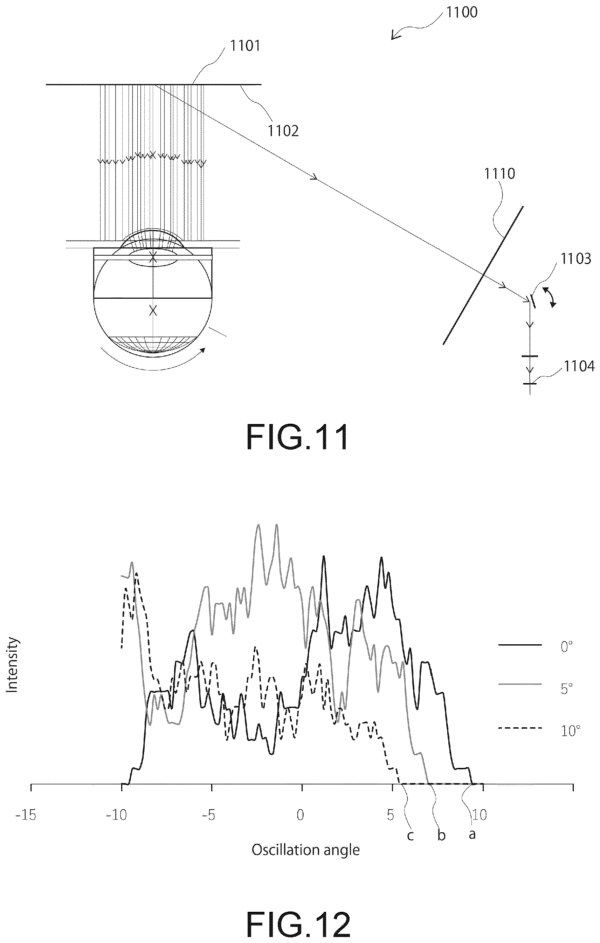

The information detection by the information detection apparatus described above in "(2) Example of First Embodiment (Information Detection Apparatus)" of the Section 1 was simulated using an OpticStudio (trademark) (manufactured by Zemax LLC.). Fig. 11 shows a configuration of the simulated apparatus. An apparatus 1100 shown in Fig. 11 includes a parallel light source 1101 as an irradiation unit, a holographic element 1102 as a light guide unit, a collimating lens 1110, a scanning mirror 1103, and a detection unit 1104.

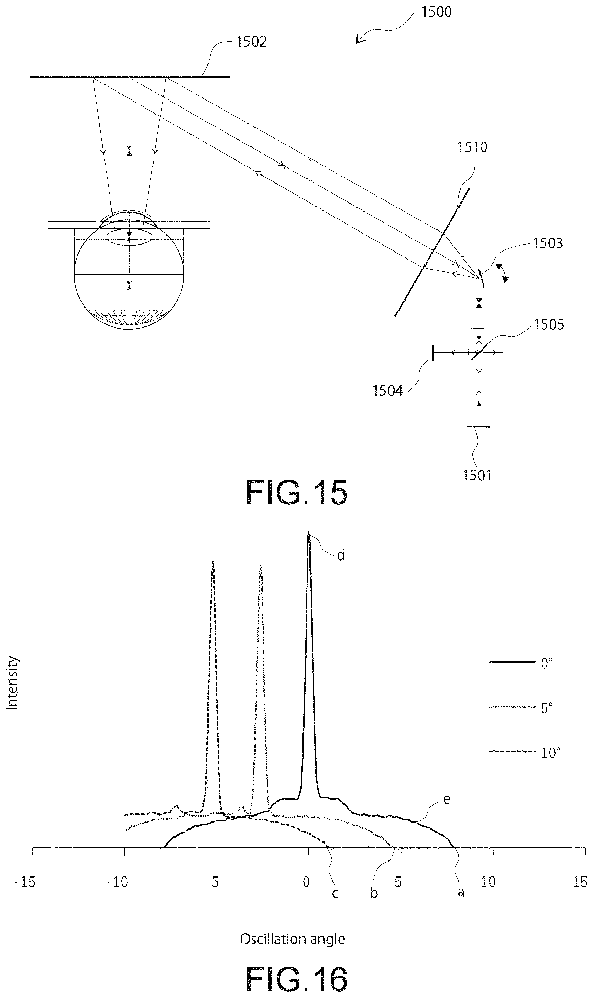

In a case where the eyeball faced the front with respect to a pupil portion and a case where a scanning oscillation angle of the scanning mirror 1103 was -10 degrees to 10 degrees, the light intensity of reflected light from the eyeball, which was detected by the detection unit 1104, was obtained by the simulation. Fig. 15 shows a relationship between the scanning oscillation angle and the light intensity.

In addition, assuming that the eyeball faced the front at 0 degrees, also in a case where the eyeball was rotated by 5 degrees or 10 degrees, the light intensity of the reflected light detected when the scanning oscillation angle was -10 degrees to 10 degrees was similarly obtained. Fig. 12 also shows a relationship between the light intensity obtained when the eyeball is rotated by 5 degrees or 10 degrees and the scanning oscillation angle.

As shown in Fig. 12, boundaries between a scanning oscillation angle when the light intensity exceeding 0 is acquired and a scanning oscillation angle when the light intensity is 0 (hereinafter, referred to as edges; indicated by a, b, and c in Fig. 12) are different between the cases where the rotation of the eyeball is 0 degrees, 5 degrees, and 10 degrees, and the edges move according to the rotation angle of the eyeball. Thus, the rotation angle, the pupil position, or the line-of-sight direction of the eyeball can be estimated on the basis of the relationship between the scanning oscillation angle and the light intensity.

In addition, comparing the cases where the rotation of the eyeball is 0 degrees, 5 degrees, and 10 degrees, the distribution of the light intensity moves in accordance with the rotation angle of the eyeball. Thus, the rotation angle, the pupil position, or the line-of-sight direction of the eyeball can be estimated also on the basis of the distribution of the light intensity with respect to the scanning oscillation angle.

The above simulation is a result obtained when the scanning oscillation angle of the scanning mirror is changed to a one-dimensional direction. Thus, by changing the scanning oscillation angle of the scanning mirror to a two-dimensional direction, information regarding the eyeball (e.g., rotation angle, pupil position, and line-of-sight direction of eyeball) can be more accurately estimated.

### (2) Example 2 (Simulation of Second Example)

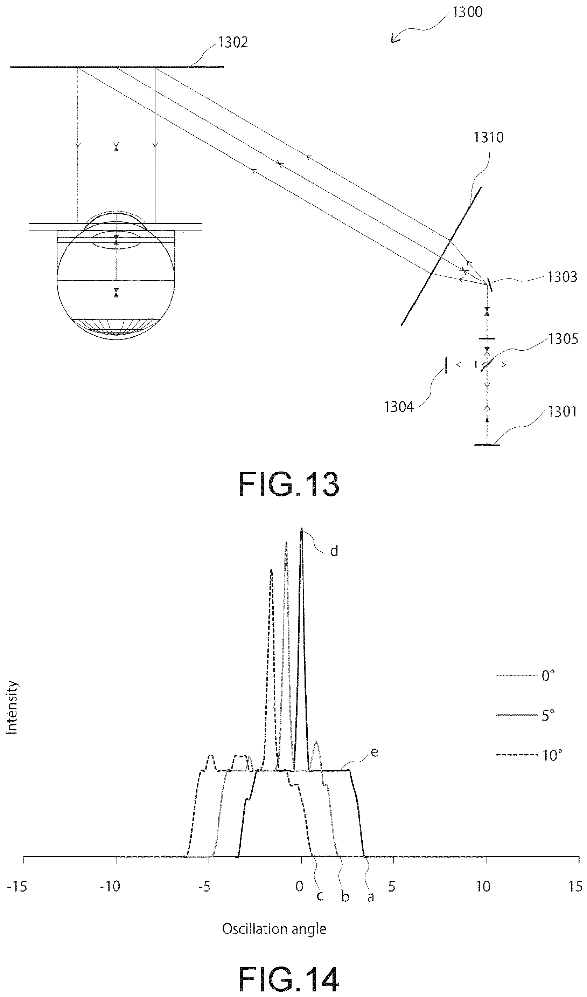

The information detection by the information detection apparatus described above in "(3) Second Example (Information Detection Apparatus)" of the Section 1 was simulated using an OpticStudio (trademark) (manufactured by Zemax LLC.). Fig. 13 shows a configuration of the simulated apparatus. An apparatus 1300 shown in Fig. 13 includes an irradiation unit 1301, a holographic element (grating) 1302 as a light guide unit, a collimating lens 1310, a scanning mirror 1303, and a detection unit 1304. In addition, the apparatus 1300 includes a half mirror 1305.

In a case where the eyeball faced the front with respect to a pupil portion and a case where a scanning oscillation angle of the scanning mirror 1303 was -10 degrees to 10 degrees, the light intensity of reflected light from the eyeball, which was detected by the detection unit 1304, was obtained by the simulation. Fig. 14 shows a relationship between the scanning oscillation angle and the light intensity.

In addition, assuming that the eyeball faced the front at 0 degrees, also in a case where the eyeball was rotated by 5 degrees or 10 degrees, the light intensity detected when the scanning oscillation angle was -10 degrees to 10 degrees was similarly obtained. Fig. 14 also shows a relationship between the light intensity obtained when the eyeball is rotated by 5 degrees or 10 degrees and the scanning oscillation angle.

As shown in Fig. 14, the edges (a, b, and c in Fig. 14) are different between the cases where the rotation of the eyeball is 0 degrees, 5 degrees, and 10 degrees, and the edges move according to the rotation angle of the eyeball. Thus, the rotation angle, the pupil position, or the line-of-sight direction of the eyeball can be estimated on the basis of the relationship between the scanning oscillation angle and the light intensity.

Comparing the cases where the rotation of the eyeball is 0 degrees, 5 degrees, and 10 degrees, the distribution of the light intensity moves according to the rotation angle of the eyeball. Thus, the rotation angle, the pupil position, or the line-of-sight direction of the eyeball can be estimated on the basis of the distribution of the light intensity with respect to the scanning oscillation angle.

In addition, the protruding peak (d in Fig. 14) can be confirmed in each of the cases where the rotation of the eyeball is 0 degrees, 5 degrees, and 10 degrees. The peaks are due to both of surface reflected light on the cornea and scattered light on the retina. Additionally, a region around the peak (e in Fig. 14) is due to the scattered light on the retina. Thus, on the basis of the peak and/or the region, the information regarding the eyeball (e.g., pupil position, line-of-sight direction, or rotation angle of eyeball) can be more accurately estimated.

In a case where the apparatus 1300 is used, the peak and/or the region can be observed, unlike the case where the apparatus 1100 of Example 1 is used. This is mainly because the beam-like light (particularly laser light) is used as the illumination light to be emitted to the eyeball. Thus, the information regarding the eyeball can be more accurately acquired by using the irradiation unit for irradiating the eyeball with beam-like light as the irradiation unit for irradiating the eyeball with light.

### (3) Example 3 (Simulation of Second Example)

The information detection by the information detection apparatus described above in "(3) Second Example (Information Detection Apparatus)" of the Section 1 was simulated using an OpticStudio (trademark) (manufactured by Zemax LLC.). Fig. 15 shows a configuration of the simulated apparatus. An apparatus 1500 shown in Fig. 15 includes an irradiation unit 1501, a holographic element (grating lens) 1502 as a light guide unit, a collimator lens 1510, a scanning mirror 1503, and a detection unit 1504. In addition, the apparatus 1500 includes a half mirror 1505.

Unlike Example 2, the holographic element 1502 is a grating lens, i.e., has a light-condensing characteristic. The illumination light from the irradiation unit is condensed toward the pupil because of the light-condensing characteristic.

In a case where the eyeball faced the front with respect to a pupil portion and a case where a scanning oscillation angle of the scanning mirror 1503 was -10 degrees to 10 degrees, the light intensity of reflected light from the eyeball, which was detected by the detection unit 1504, was obtained by the simulation. Fig. 16 shows a relationship between the scanning oscillation angle and the light intensity.

In addition, assuming that the eyeball faced the front at 0 degrees, also in a case where the eyeball was rotated by 5 degrees or 10 degrees, the light intensity detected when the scanning oscillation angle was -10 degrees to 10 degrees was similarly obtained. Fig. 16 also shows a relationship between the light intensity obtained when the eyeball is rotated by 5 degrees or 10 degrees and the scanning oscillation angle.

As shown in Fig. 16, the edges (a, b, and c in Fig. 16) are different between the cases where the rotation of the eyeball is 0 degrees, 5 degrees, and 10 degrees, and the edges move according to the rotation angle of the eyeball. Thus, the rotation angle, the pupil position, or the line-of-sight direction of the eyeball can be estimated on the basis of the relationship between the scanning oscillation angle and the light intensity.

In addition, comparing the cases where the rotation of the eyeball is 0 degrees, 5 degrees, and 10 degrees, the distribution of the light intensity moves according to the rotation angle of the eyeball. Thus, the rotation angle, the pupil position, or the line-of-sight direction of the eyeball can be estimated on the basis of the distribution of the light intensity.

The protruding peak (d in Fig. 16) can be confirmed in each of the cases where the rotation of the eyeball is 0 degrees, 5 degrees, and 10 degrees. The peaks are due to both of surface reflected light on the cornea and scattered light on the retina. Additionally, a region around the peak (e in Fig. 16) is due to the scattered light on the retina. Thus, on the basis of the peak and/or the region, the information regarding the eyeball (e.g., pupil position, line-of-sight direction, or rotation angle of eyeball) can be more accurately estimated.

In a case where the apparatus 1500 is used, the peak and/or the region can be observed, unlike the case where the apparatus 1100 of Example 1 is used. This is mainly because the beam-like light (particularly laser light) is used as the illumination light to be emitted to the eyeball. Thus, the information regarding the eyeball can be more accurately acquired by using the irradiation unit for irradiating the eyeball with beam-like light as the irradiation unit for irradiating the eyeball with light.

Although the rotation angle of the eyeball in Examples 2 and 3 is the same, the intervals of the three peaks and the intervals of the three edges observed when the apparatus 1500 is used are wider than those when the apparatus 1300 is used in Example 2. That is, even at the same rotation angle, the resolution is higher when the apparatus 1500 is used. This difference is caused by the fact that the illumination light is condensed to the eyeball because the apparatus 1500 uses a grating lens, whereas the illumination light is not condensed to the eyeball because the apparatus 1300 uses a grating. Thus, it is conceivable that the information regarding the eyeball can be acquired with higher accuracy by condensing the illumination light to the eyeball with the grating lens.

In addition, the three edges observed when the apparatus 1300 is used are sharper than those observed when the apparatus 1500 is used. For this reason, in order to acquire the information regarding the eyeball on the basis of the edges, it is considered better to use an irradiation unit that moves the illumination light straight with respect to the eyeball without condensing the illumination light.

The intensity in the region "e" (scattered light on the retina) observed when the apparatus 1300 is used is higher than that obtained when the apparatus 1500 is used. This difference may be attributed to the fact that the intensity of the scattered light on the retina detected by the detection unit is lower, because the illumination light does not travel straight toward the eyeball when the apparatus 1500 is used. Thus, in order to acquire the information regarding the eyeball on the basis of the intensity of the scattered light on the retina, it is considered better to use an irradiation unit that irradiates the eyeball with the illumination light as parallel light without concentrating the illumination light on the pupil.

### (4) Example 4 (Simulation of Example of Second Embodiment)

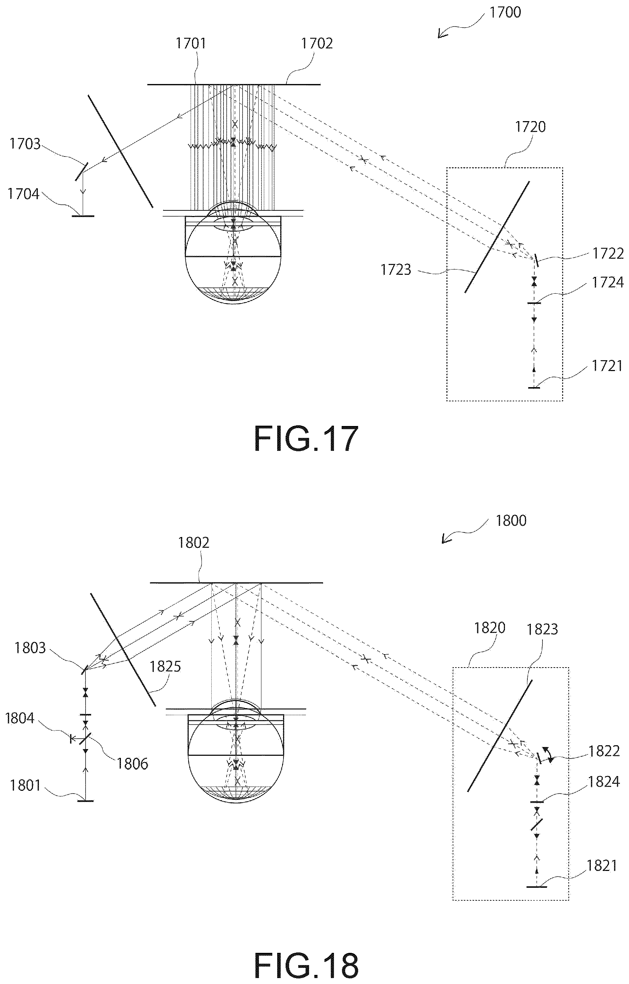

The information detection by the video projection apparatus described above in "(2) Example of Second Embodiment (Video Projection Apparatus)" of the Section 2 was simulated using an OpticStudio (trademark) (manufactured by Zemax LLC.). Fig. 17 shows a configuration of the simulated apparatus. As shown in Fig. 17, a video projection apparatus 1700 includes an irradiation unit 1701, a light guide unit 1702, a scanning mirror 1703, and a detection unit 1704. The video projection apparatus 1700 further includes a video display light irradiation unit 1720.

The video projection apparatus 1700 is an apparatus that is a specifically embodied video display light irradiation unit of the video projection apparatus 400 described above in "(2) Example of Second Embodiment (Video Projection Apparatus)". That is, the video display light irradiation unit 1720 includes an output unit 1721, a scanning mirror 1722, and a lens 1723. The output unit 1721 outputs video display light. The direction, intensity, or the like of the output video display light is adjusted by an optical system 1724. The output video display light is scanned by the scanning mirror 1722. The scanned video display light is collimated by the lens 1723 and reaches the light guide unit 1702. In such a manner, the video display light irradiation unit 1720 collimates the scanned video display light and irradiates the light guide unit 1702 with the video display light.

The light guide unit 1702 reflects the video display light such that the video display light is condensed in the vicinity of the pupil. That is, the light guide unit 1702 is a reflective grating lens for the video display light. The video display light reflected by the light guide unit 1702 travels straight through the pupil (particularly the center of the pupil) and reaches the retina. As a result, the user can recognize the video by the video display light.

In a case where the eyeball was irradiated with the video display light by the video display light irradiation unit 1720, a case where the eyeball faced the front with respect to the light guide unit, and a case where a scanning oscillation angle of the scanning mirror 1703 was -10 degrees to 10 degrees, the light intensity of the reflected light from the eyeball, which was detected by the detection unit 1704, was obtained by the simulation.

In addition, assuming that the eyeball faced the front at 0 degrees, also in a case where the eyeball was rotated by 5 degrees or 10 degrees, the light intensity of the reflected light detected when the scanning oscillation angle was -10 degrees to 10 degrees was similarly obtained.

Simulation results were similar to those obtained in Example 1 above. Therefore, it can be understood that the information regarding the eyeball can be detected also in a case where the video display light is emitted by the video display light irradiation unit.

### (5) Example 5 (Simulation of Third Example)

The information detection by the video projection apparatus described above in "(3) Third Example (Video Projection Apparatus)" of the Section 2 was simulated using an OpticStudio (trademark) (manufactured by Zemax LLC.). Fig. 18 shows a configuration of the simulated apparatus. As shown in Fig. 18, a video projection apparatus 1800 includes an irradiation unit 1801, a light guide unit 1802, a scanning mirror 1803, and a detection unit 1804. The video projection apparatus 1800 further includes a half mirror 1806 and a video display light irradiation unit 1820.

The video projection apparatus 1800 is an apparatus that is a specifically embodied video display light irradiation unit of the video projection apparatus 500 described in "(3) Third Example (Video Projection Apparatus)". That is, the video display light irradiation unit 1820 includes an output unit 1821, a scanning mirror 1822, and a lens 1823. The output unit 1821 outputs video display light. The direction, intensity, or the like of the output video display light is adjusted by an optical system 1824. The output video display light is scanned by the scanning mirror 1822. The scanned video display light is collimated by the lens 1823 and reaches the light guide unit 1802. In such a manner, the video display light irradiation unit 1820 collimates the scanned video display light and irradiates the light guide unit 1802 with the video display light.

The light guide unit 1802 reflects the video display light such that the video display light is condensed in the vicinity of the pupil. That is, the light guide unit 1802 is a reflective grating lens for the video display light. The video display light reflected by the light guide unit 1802 travels straight through the pupil (particularly the center of the pupil) and reaches the retina. As a result, the user can recognize the video by the video display light.

In addition, the video projection apparatus 1800 includes a lens 1825 between the scanning mirror 1803 and the light guide unit 1802. The illumination light scanned by the scanning mirror 1803 is collimated by the lens 1825 to reach the light guide unit 1802. The light guide unit 1802 then reflects the collimated illumination light, and the illumination light reaches the eyeball. That is, the light guide unit 1802 is a reflective grating for the illumination light.

In a case where the eyeball was irradiated with the video display light by the video display light irradiation unit 1820, a case where the eyeball faced the front with respect to the light guide unit, and a case where a scanning oscillation angle of the scanning mirror 1803 was -10 degrees to 10 degrees, the light intensity of the reflected light from the eyeball, which was detected by the detection unit 1804, was obtained by the simulation.

In addition, assuming that the eyeball faced the front at 0 degrees, also in a case where the eyeball was rotated by 5 degrees or 10 degrees, the light intensity of the reflected light detected when the scanning oscillation angle was -10 degrees to 10 degrees was similarly obtained.

Simulation results were similar to those obtained in Example 2 above. Therefore, it can be understood that the information regarding the eyeball can be detected also in a case where the video display light is emitted by the video display light irradiation unit.

### (6) Example 6 (Simulation of Fourth Example)

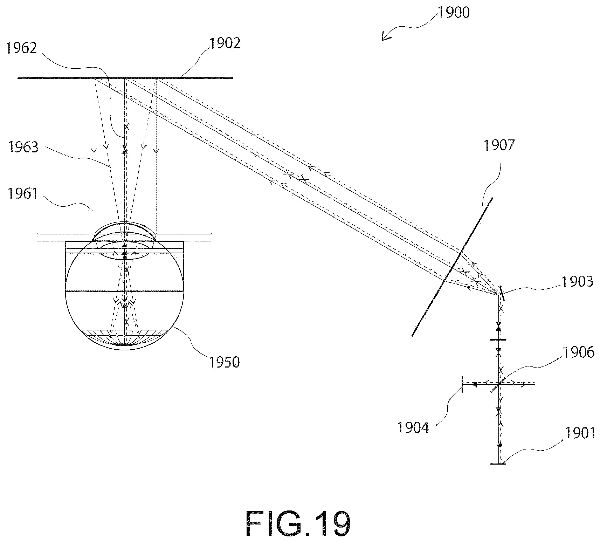

The information detection by the video projection apparatus described above in "(4) Fourth Example (Video Projection Apparatus)" of the Section 2 was simulated using an OpticStudio (trademark) (manufactured by Zemax LLC.). Fig. 19 shows a configuration of the simulated apparatus. As shown in Fig. 19, a video projection apparatus 1900 includes an irradiation unit 1901, a light guide unit 1902, a scanning mirror 1903, and a detection unit 1904. The video projection apparatus 1900 further includes a half mirror 1906 and a lens 1907.

The video projection apparatus 1900 has a configuration similar to that of the video projection apparatus 600 described above in "(4) Fourth Example (Video Projection Apparatus)".

Note that the video projection apparatus 1900 and the video projection apparatus 600 differ from each other in that the arrangement of the irradiation unit and the detection unit is reversed, and in the former, the half mirror 1906 transmits the illumination light and the video display light therethrough and reflects the reflected light. In addition, the video projection apparatus 1900 also differs in that the lens 1907 is provided between the scanning mirror 1903 and the light guide unit 1902. The illumination light scanned by the scanning mirror 1903 is collimated by the lens 1907 to reach the light guide unit 902. The light guide unit 902 then reflects the collimated illumination light, and the illumination light reaches the eyeball. That is, the light guide unit 1902 is a reflective grating for the illumination light.

In a case where the eyeball 1950 was irradiated with the video display light by the irradiation unit 1901 via the scanning mirror 1903, a case where the eyeball 1950 faced the front with respect to the light guide unit, and a case where a scanning oscillation angle of the scanning mirror 1903 was -10 degrees to 10 degrees, the light intensity of the reflected light from the eyeball 1950, which was detected by the detection unit 1904, was obtained by the simulation.

In addition, assuming that the eyeball 1950 faced the front at 0 degrees, also in a case where the eyeball 1950 was rotated by 5 degrees or 10 degrees, the light intensity of the reflected light detected when the scanning oscillation angle was -10 degrees to 10 degrees was similarly obtained.

Simulation results were similar to those obtained in Example 2 above. Therefore, it can be understood that the information regarding the eyeball can be detected also in a case where the eyeball is irradiated with the video display light by the video display light irradiation unit on the same route as the illumination light.

### Reference Signs List

- 100, 300: information detection apparatus
- 101, 301: irradiation unit
- 102, 302: light guide unit
- 103, 303: scanning mirror
- 104, 304: detection unit
- 105, 305: controller

## Claims

1. An information detection apparatus (100, 400), comprising:
an irradiation unit (101, 401) arranged to irradiate an eyeball (150) with light;
a scanning mirror (103, 403) arranged to scan reflected light from the eyeball;
a detection unit (104, 404) arranged to detect the reflected light scanned by the scanning mirror; and
a controller (105, 405) arranged to perform estimation processing for a pupil position or a line-of-sight direction of the eyeball on a basis of the reflected light detected by the detection unit,
wherein the irradiation unit is configured to irradiate the eyeball with the light directly without the scanning mirror.

2. The information detection apparatus according to claim 1, wherein
the controller is arranged to estimate the pupil position or the line-of-sight direction of the eyeball on a basis of a scanning oscillation angle of the scanning mirror and the reflected light detected by the detection unit.

3. The information detection apparatus according to claim 1, wherein
the controller is arranged to estimate the pupil position or the line-of-sight direction of the eyeball on a basis of a scanning oscillation angle of the scanning mirror and an intensity of the reflected light detected by the detection unit.

4. The information detection apparatus according to claim 1, wherein
the light for irradiating the eyeball is non-visible light.

5. The information detection apparatus according to claim 1, wherein
the light for irradiating the eyeball is infrared light.

6. A video projection apparatus, comprising:
the information detection apparatus of claim 1;
and
a video display light irradiation unit (420) arranged to irradiate the pupil position estimated by the controller with video display light such that the video display light passes through the pupil position.

7. The video projection apparatus according to claim 6, arranged to condense the video display light in a vicinity of a pupil, and to irradiate a retina with the video display light.

8. The video projection apparatus according to claim 6, arranged to irradiate the eyeball with the video display light via the scanning mirror.

9. The video projection apparatus according to claim 6, wherein
the video projection apparatus is an eyewear display.

10. An information detection method executed by an information detection apparatus, comprising:
irradiating (S102, S202) an eyeball with light by an irradiation unit (101, 401);
scanning (S103, S203) reflected light from the eyeball by a scanning mirror;
detecting (S104,S204) the reflected light scanned by the scanning mirror by a detection unit (104, 404);
performing estimation (S105, S205) processing for a pupil position or a line-of-sight direction of the eyeball on a basis of the reflected light detected by the detection unit,
wherein the irradiating the eyeball with the light is performed directly by the irradiation unit without the scanning mirror.

11. A video projection method, comprising:
the method of claim 10; and
irradiating (S206) the pupil position estimated in the estimation step with video display light such that the video display light passes through the pupil position.

## Patentansprüche

1. Informationserfassungseinrichtung (100, 400), umfassend:
eine Bestrahlungseinheit (101, 401), die konfiguriert ist, um einen Augapfel (150) mit Licht zu bestrahlen;
einen Abtastspiegel (103, 403), der angeordnet ist, um reflektiertes Licht von dem Augapfel abzutasten;
eine Erfassungseinheit (104, 404), die angeordnet ist, um reflektiertes Licht, das über den Abtastspiegel abgetastet wird, zu erfassen; und
eine Steuerung (105, 405), die angeordnet ist, um eine Verarbeitung einer Schätzung für eine Pupillenposition oder eine Blickrichtung des Augapfels auf einer Basis des reflektierten Lichts, das über die Erfassungseinheit erfasst wird, durchzuführen,
wobei die Bestrahlungseinheit konfiguriert ist, um den Augapfel ohne den Abtastspiegel direkt mit dem Licht zu bestrahlen.

2. Informationserfassungseinrichtung nach Anspruch 1, wobei
die Steuerung angeordnet ist, um die Pupillenposition oder die Blickrichtung des Augapfels auf einer Basis eines Abtastschwingungswinkels des Abtastspiegels und des reflektierten Lichts, das über die Erfassungseinheit erfasst wird, abzuschätzen.

3. Informationserfassungseinrichtung nach Anspruch 1, wobei
die Steuerung angeordnet ist, um die Pupillenposition oder die Blickrichtung des Augapfels auf einer Basis eines Abtastschwingungswinkels des Abtastspiegels und einer Intensität des reflektierten Lichts, das über die Erfassungseinheit erfasst wird, abzuschätzen.

4. Informationserfassungseinrichtung nach Anspruch 1, wobei
das Licht zum Bestrahlen des Augapfels nicht sichtbares Licht ist.

5. Informationserfassungseinrichtung nach Anspruch 1, wobei
das Licht zum Bestrahlen des Augapfels Infrarotlicht ist.

6. Videoprojektionseinrichtung, umfassend:
die Informationserfassungseinrichtung nach Anspruch 1; und
eine Videoanzeigelichtbestrahlungseinheit (420), die angeordnet ist, um die Pupillenposition, die über die Steuerung geschätzt wird, mit Videoanzeigelicht zu bestrahlen, sodass das Videoanzeigelicht durch die Pupillenposition hindurchgeht.

7. Videoprojektionseinrichtung nach Anspruch 6, die angeordnet ist, um das Videoanzeigelicht in einer Nähe einer Pupille zu kondensieren und um eine Netzhaut mit dem Videoanzeigelicht zu bestrahlen.

8. Videoprojektionseinrichtung nach Anspruch 6, die angeordnet ist, um den Augapfel mittels des Abtastspiegels mit dem Videoanzeigelicht zu bestrahlen.

9. Videoprojektionseinrichtung nach Anspruch 6, wobei
die Videoprojektionseinrichtung eine Brillenanzeige ist.

10. Informationserfassungsverfahren, das über eine Informationserfassungseinrichtung ausgeführt wird, umfassend:
Bestrahlen (S102, S202) eines Augapfels mit Licht über eine Bestrahlungseinheit (101, 401);
Abtasten (S103, S203) des reflektierten Lichts von dem Augapfel über einen Abtastspiegel;
Erfassen (S104, S204) des reflektierten Lichts, das über den Abtastspiegel abgetastet wird, über eine Erfassungseinheit (104, 404);
Durchführen der Verarbeitung der Schätzung (S105, S205) für eine Pupillenposition oder eine Blickrichtung des Augapfels auf einer Basis des reflektierten Lichts, das über die Erfassungseinheit erfasst wird,
wobei das Bestrahlen des Augapfels mit dem Licht direkt über die Bestrahlungseinheit ohne den Abtastspiegel durchgeführt wird.

11. Videoprojektionsverfahren, umfassend:
das Verfahren nach Anspruch 10; und
Bestrahlen (S206) der Pupillenposition, die in dem Schätzschritt geschätzt wird, mit Videoanzeigelicht, sodass das Videoanzeigelicht durch die Pupillenposition hindurchgeht.

## Revendications

1. Appareil de détection d'informations (100, 400) comprenant :
une unité d'irradiation (101, 401) agencée pour irradier un globe oculaire (150) avec de la lumière ;
un miroir de balayage (103, 403) agencé pour balayer la lumière réfléchie par le globe oculaire ;
une unité de détection (104, 404) agencée pour détecter la lumière réfléchie balayée par le miroir de balayage ; et
un dispositif de commande (105, 405) agencé pour effectuer un traitement d'estimation de la position de la pupille ou de la direction de la ligne de visée du globe oculaire sur la base de la lumière réfléchie détectée par l'unité de détection,
dans lequel l'unité d'irradiation est configurée pour irradier le globe oculaire avec la lumière directement sans le miroir de balayage.

2. Appareil de détection d'informations selon la revendication 1, dans lequel
le dispositif de commande est agencé pour estimer la position de pupille ou la direction de ligne de visée du globe oculaire sur la base d'un angle d'oscillation de balayage du miroir de balayage et de la lumière réfléchie détectée par l'unité de détection.

3. Appareil de détection d'informations selon la revendication 1, dans lequel
le dispositif de commande est agencé pour estimer la position de pupille ou la direction de ligne de visée du globe oculaire sur la base d'un angle d'oscillation du miroir de balayage et d'une intensité de la lumière réfléchie détectée par l'unité de détection.

4. Appareil de détection d'informations selon la revendication 1, dans lequel
la lumière permettant d'irradier le globe oculaire est une lumière non visible.

5. Appareil de détection d'informations selon la revendication 1, dans lequel
la lumière permettant d'irradier le globe oculaire est une lumière infrarouge.

6. Appareil de projection vidéo comprenant :
l'appareil de détection d'informations selon la revendication 1 ; et
une unité d'irradiation de lumière d'affichage vidéo (420) agencée pour irradier la position de pupille estimée par le dispositif de commande avec de la lumière d'affichage vidéo de telle sorte que la lumière d'affichage vidéo passe à travers la position de pupille.

7. Appareil de projection vidéo selon la revendication 6, agencé pour condenser la lumière d'affichage vidéo à proximité d'une pupille et pour irradier une rétine avec la lumière d'affichage vidéo.

8. Appareil de projection vidéo selon la revendication 6, agencé pour irradier le globe oculaire avec la lumière d'affichage vidéo par l'intermédiaire du miroir de balayage.

9. Appareil de projection vidéo selon la revendication 6, dans lequel l'appareil de projection vidéo est un écran de lunettes.

10. Procédé de traitement d'informations exécuté par un appareil de détection d'informations, comprenant :
l'irradiation (S102, S202) d'un globe oculaire avec de la lumière par une unité d'irradiation (101, 401) ;
le balayage (S103, S203) de la lumière réfléchie à partir du globe oculaire par un miroir de balayage ;
la détection (S104, S204) de la lumière réfléchie balayée par le miroir de balayage par une unité de détection (104, 404) ;
le fait d'effectuer un traitement d'estimation (S105, S205) de la position de pupille ou une direction de ligne de visée du globe oculaire sur la base de la lumière réfléchie détectée par l'unité de détection,
dans lequel l'irradiation du globe oculaire avec la lumière est effectuée directement par l'unité d'irradiation sans le miroir de balayage.

11. Procédé de projection vidéo, comprenant :
le procédé selon la revendication 10 ; et
l'irradiation (S206) de la position de pupille estimée, lors de l'étape d'estimation avec la lumière de l'écran vidéo de sorte que la lumière d'affichage vidéo passe par la position de pupille.
